# EUROPÄISCHE PATENTANMELDUNG

(11) **EP 2 319 802 A1**
(43) Veröffentlichungstag der Anmeldung: **11.05.2011**
(21) Anmeldenummer: 09014032.8
(22) Anmeldetag: 10.11.2009
(51) Int. Cl.: C01F 7/00, C01F 7/16

(54) **Mischmetalloxyde und deren Verwendung als Katalysatoren für die Alkoxylierung**

(71) Anmelder: Cognis IP Management GmbH, 40589 Düsseldorf (DE)
(72) Erfinder: Lercher, Johannes, 85521 Ottobrunn (DE); Mueller, Thomas, 52062 Aachen (DE); Raths, Hans-Christian, 40789 Monheim (DE); Bezen, Manuela, 80803 München (DE); Kruppa, Thomas, 47803 Krefeld (DE)

(57) **Zusammenfassung**

Mischmetalloxide der allgemeinen Formel (I)

[MgₓZn_{y}]₄Na_{z}AlOₙ (I)

in der x die Wert von 1 bis 4, y die Werte von 0 bis 3 und z die Werte von 0 bis 25 einnehmen kann, wobei n eine Zahl von 5 bis 18 wiedergibt, wobei die Mischmetalloxide weiterhin gekennzeichnet sind durch ein Molverhältnis von Mg zu Al im Bereich von 3,5 bis 4,5 : 1 eignen sich als Katalysatoren bei der Alkoxylierung von Fettalkoholen, Alkylphenolen, Fettaminen, Fettsäuren und deren Amiden, Fettsäureestern, Mercaptanen und Imidazolinen.

## Beschreibung

Die vorliegende Anmeldung betrifft bestimmte Mischmetalloxide und deren Verwendung als Katalysatoren insbesondere bei der Alkoxylierung von organischen Substraten, die mindestens ein acides Wasserstoffatom enthalten.

Mischmetalloxide, das heißt Oxide die mehrere unterschiedliche Metallkationen enthalten sind an sich bekannte Katalysatoren für verschiedene chemische Reaktionen, darunter auch der Umsetzung von organischen Substraten mit Alkoxiden, wie insbesondere Ethylenoxid oder Propylenoxid. Aus der DE 39 14 131 A1 sind z.B. Hydrotalcite mit der allgemeinen Formel MgₓAI(OH)_{y}(CO₃)_{z} H₂O bekannt, die in kalzinierter Form die Reaktion von Fettsäuren oder Alkanolen mit Ethylenoxid katalysieren, wobei die Verteilung der so hergestellten homologen Ethoxylate sehr eng ist (sog. "narrow range ethoxylates"). Aus der DE 199 09 272 A1 ist bekannt, dass sich unterschiedliche alkoxylierte Tenside mit Hilfe von Hydrotalciten als heterogene Katalysatoren erhalten lassen. Die DE 44 46 064 A1 beschreibt ein Verfahren zur Herstellung von Polyoxyalkylenalkylether-Fettsäureestern mit einem Mg-Al Mischmetalloxid-Katalysator vom Typ des Hydrotalcits.

Wegen der großen Bedeutung, die die Alkoxylierung bei der Herstellung von Tensiden hat, besteht aber ein ständiges Bedürfnis weitere geeignete Katalysatoren mit vorteilhaften Eigenschaften (z.B. höhere Effizienz, oder bessere Steuerung des Produktspektrum der Alkoxylierung) aufzufinden.

Es wurde gefunden, dass ausgewählte Mischmetalloxide vorteilhafte Eigenschaften aufweisen.

Ein erster Gegenstand der vorliegenden Anmeldung sind Metallmischoxide, gekennzeichnet durch die allgemeine Formel (I)

[MgₓZn_{y}]₄Na_{z}AlOₙ (I)

in der x die Werte von 1 bis 4, y die Werte von 0 bis 3 und z die Werte von 0 bis 25 einnehmen kann, wobei n eine Zahl von 5 bis 18 wiedergibt, wobei die Mischmetalloxide weiterhin gekennzeichnet sind durch ein Molverhältnis von Mg zu Al im Bereich von 3,5 bis 4,5 : 1.

Die Mischmetalloxide gemäß der vorliegenden Erfindung enthalten zwingend Magnesium und Aluminium sowie optional Zink und/oder Natrium nebeneinander. Dabei gilt, dass Mischmetalloxide die Zn und Na enthalten bevorzugt sein können. Insbesondere sind solche Mischmetalloxide der allgemeinen Formel (I) bevorzugt, bei denen das Molverhältnis zwischen Mg und Al im Bereich von 3,9 : 1 bis 4,1 : 1 und insbesondere 4,0 : 1 beträgt.

Sofern Zn enthalten ist gilt, dass das Molverhältnis zwischen der Summe aus Mg und Zn zu Al vorzugsweise im Bereich von 3,5 : 1 bis 4,5 :1 liegt und besonders vorzugsweise 3,6 : 1 bis 3,8 : 1 beträgt.

Weiterhin sind aber solche Mischmetalloxide der allgemeinen Formel (I) bevorzugt, die Na, vorzugsweise Na und Zn enthalten. Es gilt weiterhin für die Na-haltigen Mischmetalloxide als bevorzugte Ausfi,ihrungsform wenn das Molverhältnis zwischen Aluminium und Natrium im Bereich von 25 : 1 bis 1 : 1 liegt, vorzugsweise im Bereich von 10 : 1 bis 5 : 1 liegt.

Es gehört aber auch zu den bevorzugten Ausführungsformen der vorliegenden Lehre Na-freie Mischmetalloxide gemäß der allgemeinen Formel (I) bereit zustellen. Solche Mischmetalloxide können beispielsweise durch Soxhlet-Extraktion von Na-haltigen Oxiden mittels Ethanol als Extraktionsmittel hergestellt werden.

Die Mischmetalloxide gemäß der vorliegenden technischen Lehre weisen spezifische Oberflächen (gemessen nach der BET-Methode) von mindesten 45 m²/g, vorzugsweise von 50 bis 200 m²/g auf. Ein weiteres Charakteristikum für Mischmetalloxide des vorliegenden Typs ist das Verhältnis von tetraedrischem zu oktaedrischem Aluminium in der Struktur. Vorteilhafte Werte liegen zwischen 0,01 und 0,80 wobei der Bereich von 0,1 zu 0,70 bevorzugt sein kann. Messverfahren finden sich in der Veröffentlichung von Lima et al., Microporous and Mesoporous Materials 107 (2008) 240-246.

Mischmetalloxide können nach vielfältigen dem Fachmann bekannten Verfahren synthetisiert werden, so z.B. durch Extrusion, Fällungsverfahren, einer Imprägnierung aber vorzugsweise durch ein Sol-Gel-Verfahren, bei dem Metallnitrate in basischer Lösung gefällt, getrocknet und dann kalziniert werden.

Ein weiteres Verfahren wird in der oben bereits genannten Veröffentlichung von Lima et al. beschrieben wobei gemäß dieser Schrift eine feste Mischung aus Magnesiumnitrat, Aluminiumnitrat und Natriumcarbonat mit Saccharose in Wasser suspendiert und dann bei 80°C zur Reaktion gebracht werden bis das Wasser verdampft ist. Anschließend wird das Zwischenprodukt kalziniert.

Es wurde nun gefunden, dass auf den Zusatz von Zucker verzichtet werden kann. Ein weiterer Gegenstand der Anmeldung betrifft daher ein vereinfachtes Verfahren zur Herstellung von Mischmetalloxiden gemäß der Beschreibung in Anspruch 1, wobei man
(a) Magensiumnitrat ggf. in Abmischung mit Zinknitrat, mit Aluminiumnitrat in wässeriger Lösung in Gegenwart von Carbonat- oder Hydrogencarbonationen bei pH-Werten von 8 bis 10 und Temperaturen von 60 bis 90°C zur Reaktion bringt, anschließend den erhaltenen Niederschlag abfiltriert und
(b) den Niederschlag bei Temperaturen von mindestens 60°C unter erhöhtem Druck weiter reagieren lässt und
(c) anschließend den getrockneten Niederschlag aus (c) bei mindestens 500°C kalziniert.

Im ersten Schritt (a) reagieren die Salze zu Hydroxiden oder Hydrotalciten. Der Zusatz von Carbonat- bzw. Hydrogencarbonationen dient der pH-Regulation. Der pH-Wert beträgt während des Reaktionsschritts (a) vorzugsweise 8 bis 12 und insbesondere 9 bis 11, wobei ein pH von 10 besonders vorteilhaft sein kann. Im Schritt (b) wird das Wasser entfernt, so dass ein feuchter Niederschlag zurückbleibt, der dann unter Druck weiter erhitzt wird. Die Reaktion im Schritt (b) erfolgt bei mindestens 60°C, wobei aber auch Temperaturen von 60 bis 150°C, vorzugsweise aber von 75 bis 85°C eingestellt werden können. Die Umsetzung erfolgt wegen des erhöhten Drucks vorzugsweise in einem Autoklaven, wobei hier Zeiten von 3h bis 24 h eingestellt werden können. Der während der Reaktion aufgebaute Druck kann (bei 80°C) im Bereich von 1,5 bis 150 kPa, vorzugsweise aber 100 bis 145 kPa betragen.

Es kann vorteilhaft sein, den Niederschlag aus dem Schritt (b) zunächst für ca. 1 bis 5 h bei Temperaturen von 60 bis 80 °C zu altern, bevor dann Schritt (c) durchgeführt wird. Die Umsetzung im Schritt (b) stellt eine Hydrothermalsynthese dar, bei der die ausgefallenen Hydroxide bzw. Hydrotalcite aus dem Verfahrensschritt (a) bei erhöhter Temperatur und Druck weiterreagieren, wobei der Schritt (b) zu einer höheren spezifischen Oberfläche führt.

Anschließend wird im Schritt (c) kalziniert. Unter Kalzinierung (oder Calcinierung) wird das Erhitzen fester Materialien (Brennen) in sauerstoffhaltiger Atmosphäre bis zu einem bestimmten Zersetzungsgrad verstanden, wodurch z. B. bei Soda, Gips und anderen Stoffen das Kristallwasser ganz oder teilweise entfernt wird. Im vorliegenden Fall führt die Kalzinierung durch Abspaltung von Wasser und von CO₂ zur Bildung der erfindungsgemäßen Mischmetalloxide. Der Kalzinierungsschritt erfolgt vorzugsweise unter einer Atmosphäre aus synthetischer Luft und wird bei Temperaturen von 500 °C bis 800 °C, vorzugsweise aber bei 600 °C durchgeführt. Hier bilden sich aus den Hydroxiden und Hydrotalciten die erfindungsgemäßen Mischmetalloxide. Es kann vorteilhaft sein, den Niederschlag vor der Kalzinierung durch Gefriertrocknung von Lösungsmittelresten zu befreien. Die Kalzinierung nimmt typischerweise 2 bis 10 h in Anspruch.

Die Mischmetalloxide gemäß der obigen allgemeinen Formel (I) eignen sich vorteilhaft als Katalysatoren für die Alkoxylierung von organischen Substraten mit aktiven H-Atomen. Bei der Alkoxylierung wird ein Oxiran oder dessen Homologen, vorzugsweise Ethylenoxid, Propylenoxid oder Butylenoxid mit einem Substrat, welches mindestens ein acides Wasserstoffatom enthält umgesetzt. Geeignete Substrate mit acidem Wasserstoff-Atom sind Fettalkohole, Alkylphenole, Fettamine, Fettsäuren und deren Amide, Fettsäureester, Mercaptane und Imidazoline.

Die Produkte der technisch, bei Temperaturen zwischen 120 und 220°C unter Druck (ca. 100-500 kPa) ausgeführten Reaktionen sind lineare Ether bzw. Polyether, die an einem Kettenende eine Alkoxyd-Gruppe, am anderen eine vom Ausgangsprodukt abhängige funktionelle Gruppe tragen.

Die Verwendung der oben beschriebenen Mischmetalloxide der allgemeinen Formel (I) für die Alkoxylierung von organische Substraten, ausgewählt aus der Gruppe der Fettalkohole, Alkylphenole, Fettamine, Fettsäuren und deren Amide, Fettsäureester, Mercaptane und Imidazoline ist ein weitere Gegenstand der Anmeldung. Es können unterschiedliche Mischmetalloxide gemäß der obigen Beschreibung entweder alleine oder in beliebigen Mischungen untereinander erfindungsgemäß Verwendung finden. Dabei ist es bevorzugt, dass als Alkoxide Ethylenoxid und/oder Propylenoxid ausgewählt sind und als Substrate Fettalkohole, Fettsäuren oder Fettsäureamide ausgewählt werden. Die Alkoxylierung von Fettalkoholen ist besonders bevorzugt. Die Mischmetalloxide der allgemeinen Formel (I) werden dabei vorzugsweise in Mengen von 0,1 bis 20 Gew.-%, bezogen auf das Gewicht der Substrate eingesetzt.

Auffallend ist, dass die Verwendung von Na-haltigen Mischmetalloxiden der allgemeinen Formel (I) zu Alkoxylaten mit breiterer Verteilung der Homologen führt, als wenn Mischmetalloxide der Formel (I) eingesetzt werden, die Na-frei sind. Somit eignen sich die Mischmetalloxide zur gezielten Steuerung des Verteilungsspektrums von Homologen in Alkoxylierungsverfahren. Vorzugsweise werden die Mischmetalloxide dazu verwendet in Alkoxylierungsverfahren (vorzugsweise mit Ethylenoxid oder Propylenoxid) Produkte mit einer breiten Verteilung der Homologen zu erhalten. Mit einer breiten Verteilung ist dabei eine Verteilung nach Schulz-Flory gemeint, wohingegen eine enge Homologenverteilung einer Poisson-Verteilung folgt.

Um die Mischmetalloxide als Katalysatoren einsetzen zu können, können diese entweder als Pulver der Reaktionsmischung beigegeben werden, oder vorzugsweise mit Binder und Trägermaterialien (z.B. Methylcellulose) zu Formkörpern gepresst werden, die dann im Alkoxylierungsreaktor eingesetzt werden können.

Feste Katalysatoren, die aus einem Träger, einem Binder und einem Mischmetalloxid gemäß der obigen Beschreibung sowie optional weiteren Zusatzstoffen bestehen sind daher ebenfalls Gegenstand der vorliegenden Anmeldung. Als Träger können z.B. anorganische Salze, z.B. Sulfate aber auch Silikate oder keramische Werkstoffe eingesetzt werden, aber auch organische Träger, z.B. Methylcellulose sind geeignete Substanzen. Vorzugsweise enthalten solche Katalysatoren mindestens 50 Gew.-% an Mischmetalloxiden, insbesondere aber zwischen 55 und 90 Gew.-%.

### Beispiele

### Herstellung der Metallmischoxide

Ein Mischoxid (1) mit einem Mg/Al-Verhältnis von 4 : 1 wurde wie folgt hergestellt: 123,08 g (0,48 mol) Mg(NO₃)₂ 6 H₂O und 45,01 g (0,12 mol) Al(NO₃)₃ 9 H₂O wurden in 600 mL Wasser (bidest.) gelöst. Eine weitere Lösung wurde aus 48 g (1,2 mol) NaOH und 5,04 g (0,06 mol) NaHCO₃ in 600 mL Wasser (bidest.) zubereitet. Beide Lösungen wurden tropfenweise bei 70 °C zu 400 mL Wasser (bidest.) hinzu gegeben, dessen pH-Wert auf 9 konstant gehalten wurde. Der sich bildende Niederschlag wurde bei 80 °C für 3 h gealtert. Anschließend wurde der Niederschlag abfiltriert und bei 80 °C 16 h in einem Autoklav behandelt. Der Niederschlag wurde gewaschen, das Wasser durch Gefriertrocknung entfernt und anschließend bei 600 °C 6 h in synthetischer Luft kalziniert. Ein Teil des so erhaltenen Mischoxides wurde durch Soxhletextraktion vollständig von Na befreit (1a).

Es wurden weiterhin Na-modifizierte Mischoxide hergestellt, wobei gemäß der Verfahrensvorschrift zu (1) vorgegangen wurde. Die Variation des Na-Gehalts wurde gezielt durch das Waschen mit unterschiedlichen Mengen an Wasser (150 und 350 mL) bzw. durch den Verzicht des Waschgangs erhalten.

Zink-modifizierte Mischoxide wurden analog zu (1) hergestellt, wobei unterschiedliche Verhältnisse von Mg zu Zn eingestellt wurden. In der Tabellen 1 und 2 sind die physikochemischen Parameter und die Zusammensetzung der Mischmetalloxide wiedergegeben. Während der Reaktion wird ein erhöhter Druck, vorzugsweise zwischen 1,5 bis 10 bar eingestellt. Die Reaktionstemperatur wird vorzugsweise auf Werte von 60 bis 200°C und vorzugsweise von 100 bis 180 °C insbesondere 120 bis 180 °C eingestellt.

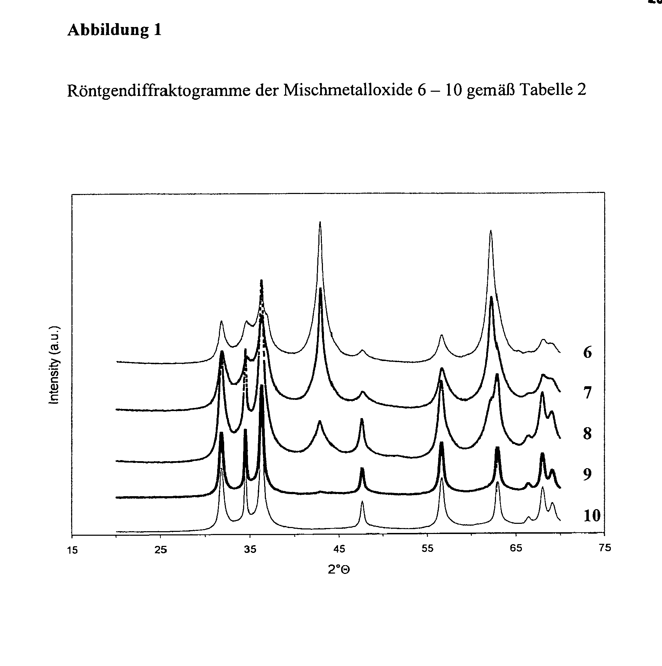

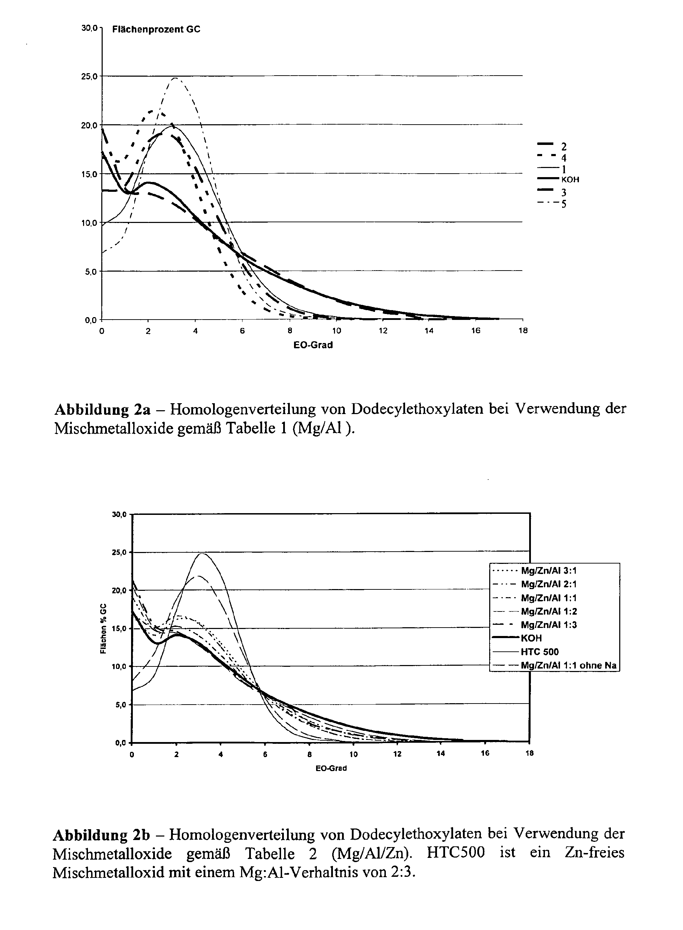

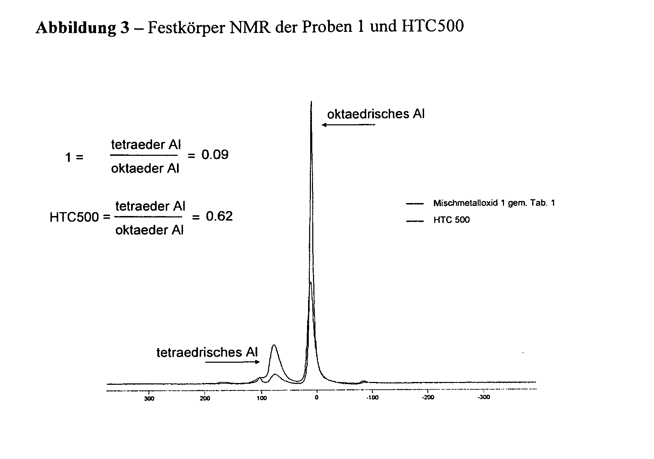

In der Abbildung 1 sind die Röntgendiffraktogramme der Mischmetalloxide 6 bis 11 wiedergegeben. Die Messung erfolgte mit einem Diffraktometer Xpert Pro, Fa. PANanalytical B.V. In der Abbildung 2 sind zwei NMR-Spektren der erfindungsgemäßen Mischmetalloxide wiedergegeben, aus denen sich das Verhältnis von oktaedrischem zu tetraedrischem Aluminium berechnen lässt.

### Methode zur Bestimmung der spezifischen Oberfläche (BET):

Die Bestimmung der spezifischen Oberfäche erfolgt mit Hilfe der BET-Methode. Diese ist nach ihren Entwicklern Brunauer, Emmett und Teller benannt. Sie beruht darauf, dass ein adsorbierbarer Stoff (Adsorptiv) in der Gasphase die Oberfläche eines festen Körpers (Adsorbens) mit einem Adsorptionsfilm (Adsorpt) bedecken kann.

Die spezifische Oberfläche und das Porenvolumen der Katalysatoren wurden konkret durch Phsisorption von N₂ bei 77 K bestimmt. Es wurde dazu ein PMI automatisiertes BET-Sorptometer verwendet.

**Tabelle 1 : Mischmetalloxide Zn-frei**

| **Nr.** | **Spezifische Oberfläche¹⁾** | **Mg:Al** | **Mg:Zn** | **Mg-Gehalt** | **Al-Gehalt** | **Na-Gehalt** | **Mg-Gehalt** | **Al-Gehalt** | **Na-Gehalt** |
|---|---|---|---|---|---|---|---|---|---|
| | [m²/g] | | | [µmol/g] | [mol/g] | [µmol/g] | [wt.-%] | [wt.-%] | [wt.-%] |
| **1** | 182 | 3,9 | - | 16,7 | 4,30 | 0,50 | 40,6 | 11,7 | 1,14 |
| **2** | 128 | 3,8 | - | 16,5 | 4,40 | 1,40 | 40,1 | 11,8 | 3,21 |
| **3** | 29 | 3,9 | - | 9,40 | 2,40 | 6,10 | 22,80 | 6,61 | 14,00 |
| **4** | 105 | 3,8 | - | 1,522 | 0,404 | 0,24 | 37 | 10,9 | 5,45 |
| **5** | 129 | 3,5 | - | 1,74 | 0,5 | 0,09 | 42,3 | 12,3 | 1,99 |

| | | | | | | | | | |
|---|---|---|---|---|---|---|---|---|---|
| ¹⁾ mittels N₂-BET bzw. Hg₂-Porosiät | | | | | | | | | |

**Tabelle 2: Mischmetalloxide Zn-haltig**

| **Nr.** | **Metalloxid¹⁾** | S**pezifische Oberfläche** ^{1,2)} | **Mg+Zn:Al** | **Mg:Zn** | **Mg-Gehalt** | **Al-Gehalt** | **Na-Gehalt** | **Zn-Gehalt** | **Mg-Gehalt** | **Al-Gehalt** | **Na-Gehalt** | **Zn-Gehalt** |
|---|---|---|---|---|---|---|---|---|---|---|---|---|
| | | [m²/g] | | | [mmol/g] | [mmol/g] | [mmol/g] | [mmol/g] | [wt.-%] | [wt.-%] | [wt.-%] | [wt.-%] |
| **6** | [MgZn]Al [3:1]4:1 | 123 | 3,8 | 2,5 | 10,66 | 3,96 | - | 4,19 | 25,9 | 10,7 | | 27,4 |
| **7** | [MgZn]Al [2:1]4:1 | 100 | 3,8 | 1,7 | 8,76 | 3,68 | - | 5,14 | 21,3 | 9,9 | | 33,6 |
| **8** | [MgZn]Al [1:1]4:1 | 111 | 3,9 | 0,8 | 5,97 | 3,37 | 0,14 | 7,07 | 14,5 | 9,1 | 0,3 | 46,2 |
| **9** | [MgZn]Al [1:2]4:1 | 75 | 3,8 | 0,4 | 3,10 | 2,80 | 1,00 | 7,50 | 7,6 | 7,6 | 2,3 | 49.1 |
| **10** | [MgZn]Al [1:3]4:1 | 64 | 3,7 | 0,2 | 2,10 | 2,90 | 0,70 | 8,60 | 5,0 | 7,7 | 1,6 | 56,2 |
| **11** | [MgZn]Al [3:1]4:1 | 85 | 3,9 | 2,9 | 9,75 | 3,41 | 1,60 | 3,38 | 23,7 | 9,2 | 3,7 | 22,1 |
| **12** | [MgZn]Al [2:1]4:1 | 94 | 3,8 | 1,9 | 8,60 | 3,45 | 1,60 | 4,47 | 20,9 | 9,3 | 3,7 | 29,2 |
| **13** | [MgZn]Al [1:1]4:1 | 78 | 3,7 | 0,9 | 5,72 | 3,23 | 1,20 | 6,39 | 13,9 | 8,7 | 2,7 | 41,8 |
| **14** | [MgZn]Al [1:2]4:1 | 41 | 3,7 | 0,4 | 3,33 | 3,00 | 0,90 | 7,74 | 8,1 | 8,1 | 2,0 | 50,6 |
| **15** | [MgZn]Al [1:3]4:1 | 48 | 3,6 | 0,3 | 2,29 | 3,02 | 1,30 | 8,61 | 5,6 | 8,2 | 3,1 | 56,3 |

| | | | | | | | | | | | | |
|---|---|---|---|---|---|---|---|---|---|---|---|---|
| ² Die Zahlen in der eckigen Klammer geben das molare Verhältnis von Mg und Zn wieder; die zahlen außerhalb der eckigen Klammer geben das molare Verhältnis von Al zu Mg und Zn wieder. | | | | | | | | | | | | |

### Testung der katalytischen Eigenschaften

Die Mischmetalloxide wurden jeweils zur Eignung als Alkoxylierungskatalysator für die Ethoxylierung von Dodecanol untersucht: Zum Test der katalytischen Aktivität wurden jeweils 5 g des Festkörperkatalysators in 580g (3,1 Mol) Dodecanol suspendiert. Diese Suspension wurde in einen Autoklaven überführt, der anschließend gründlich mit Stickstoff gespült und für 30 min bei 120°C auf 10 mbar evakuiert wurde. Anschließend wurde das Vakuum mit Stickstoff aufgehoben und die gewünschte Menge an Ethylenoxid (hier 9,3 Mol = 409g) mit Stickstoff überlagert bei max. 5 bar in den Autoklaven dosiert, wobei die Temperatur 170 - 180°C nicht überschritt. Nach Abschluss der Dosierung und bei konstantem Innendruck wurde 60 min nachgerührt. Anschließend wurde zur Entfernung restlichen Ethylenoxids für weitere 30 min bei 120°C evakuiert. Danach wurde mit Stickstoff belüftet und unter Rühren auf 60-80°C abgekühlt und über einen Seitz-Druckfilter filtriert.

Die gebildeten Ethoxylate wurden silyliert und gaschromatgraphisch qualitativ und quantitativ untersucht, um den Anteil der jeweiligen Homologen für jeden der untersuchten Mischmetalloxidkatalysatoren zu bestimmen.

In der Abbildung 2a ist die Produktverteilung für die Mischmetalloxide der Tabelle 1, in der Abbildung 2b die Verteilung der Mischmetalloxide der Tabelle 2 graphisch wiedergeben.

Man erkennt, dass die Na-haltigen Mischmetalloxide zu breiten Verteilungen der Homologen führen, wohingegen die Abwesenheit von Natrium in den Mischmetallen zu einer deutlich engeren Verteilung der Ethoxylate führt.

## Patentansprüche

1. Metallmischoxid, **gekennzeichnet durch** die allgemeine Formel (I)
[MgₓZn_{y}]₄Na_{z}AlOₙ (I)
in der x die Wert von 1 bis 4, y die Werte von 0 bis 3 und z die Werte von 0 bis 25 einnehmen kann, wobei n eine Zahl von 5 bis 18 wiedergibt, wobei die Mischmetalloxide weiterhin **gekennzeichnet sind durch** ein Molverhältnis von Mg zu Al im Bereich von 3,5 bis 4,5 : 1.

2. Mischmetalloxide nach Anspruch 1, **dadurch gekennzeichnet, dass** das Molverhältnis zwischen Mg und Al im Bereich von 3,9 : 1 bis 4,1 : 1 und insbesondere 4,0 : 1 beträgt.

3. Mischmetalloxide nach einem oder mehreren der Ansprüche 1 bis 2, **dadurch gekennzeichnet, dass** das Molverhältnis zwischen der Summe aus Mg und Zn zu Al 3,5 : 1 bis 4,5 :1 und vorzugsweise 3,6 : 1 bis 3,8 : 1 beträgt.

4. Mischmetalloxide nach einem oder mehreren der Ansprüche 1 bis 3, **dadurch gekennzeichnet, dass** deren spezifische Oberfläche mindesten 45 m²/g, vorzugsweise von 50 bis 200 m²/g beträgt.

5. Mischmetalloxide nach einem oder mehreren der Ansprüche 1 bis 4, **dadurch gekennzeichnet, dass** sie Natrium enthalten.

6. Mischmetalloxide nach einem oder mehreren der Ansprüche 1 bis 4, **dadurch gekennzeichnet, dass** sie frei von Natrium sind

7. Mischmetalloxide nach einem oder mehreren der Ansprüche 1 bis 6, **dadurch gekennzeichnet, dass** das Molverhältnis zwischen Aluminium und Natrium im Bereich von 25 : 1 bis 1 : 1 liegt, vorzugsweise im Bereich von 10 : 1 bis 5 : 1.

8. Mischmetalloxide nach einem oder mehreren der Ansprüche 1 bis 7, **dadurch gekennzeichnet, dass** das Verhältnis zwischen tetraedrischem und oktaedrischem Aluminium im Bereich von 0,01 bis 0,80, vorzugsweise von 0,1 bis 0,70 liegt.

9. Verfahren zur Herstellung von Mischmetalloxiden gemäß der Beschreibung in Anspruch 1, **dadurch gekennzeichnet, dass** man
(a) Magnesiumnitrat ggf. in Abmischung mit Zinknitrat mit Aluminiumnitrat in wässeriger Lösung in Gegenwart von Carbonat- oder Hydrogencarbonationen bei pH-Werten von 8 bis 10 und Temperaturen von 60 bis 90 °C zur Reaktion bringt, anschließend
(b) den erhaltenen Niederschlag abfiltriert und
(c) den Niederschlag in Wasser löst und bei Temperaturen von mindestens 60 °C unter erhöhtem Druck weiter reagieren lässt, und
(d) anschließend den getrockneten Niederschlag bei mindestens 500 °C kalziniert.

10. Verwendung von Mischmetalloxiden gemäß der Beschreibung der Ansprüche 1 bis 8 als Katalysator für die Alkoxylierung von organische Substraten, ausgewählt aus der Gruppe der Fettalkohole, Alkylphenole, Fettamine, Fettsäuren und deren Amide, Fettsäureester, Mercaptane und Imidazoline.

11. Verwendung nach Anspruch 10, **dadurch gekennzeichnet, dass** als Alkoxide Ethylenoxid und/oder Propylenoxid ausgewählt sind und als Substrate Fettalkohole, Fettsäuren oder Fettsäureamide ausgewählt werden.

12. Verwendung nach Anspruch 10 oder 11, **dadurch gekennzeichnet, dass** die Mischmetalloxide als Katalysatoren zur Herstellung von Alkoxylaten mit einer breiten Verteilung der Homologen verwendet werden.

13. Fester Katalysator, **dadurch gekennzeichnet, dass** er aus einem Träger, Binder und Mischmetalloxiden gemäß dem Anspruch 1 sowie optional weiteren Zusatzstoffen besteht.
